# EUROPEAN PATENT APPLICATION

(11) **EP 3 176 182 A1**
(43) Date of publication of application: **07.06.2017**
(21) Application number: 15894761.4
(22) Date of filing: 13.10.2015
(51) Int. Cl.: C07K 16/28, C12N 15/13, C12N 15/63, A61K 39/395, A61P 35/00, A61P 37/00, G01N 33/577

(54) **ANTI-PD-1 MONOCLONAL ANTIBODY AND OBTAINING METHOD THEREFOR**

(30) Priority: 09.06.2015 CN 201510312910
(71) Applicant: Beijing Dongfang Biotech Co., Ltd, Beijing 100176 (CN); Beijing Jingyitaixiang Technology Development Co., Ltd., Beijing 100176 (CN)
(72) Inventor: ZHOU, Haiping, Beijing 100176 (CN); LI, Xiaomin, Beijing 100176 (CN); ZHOU, Junjie, Beijing 100176 (CN); PEI, Shuang, Beijing 100176 (CN); ZAN, Yanlu, Beijing 100176 (CN); BAI, Yi, Beijing 100176 (CN); BAI, Xianhong, Beijing 100176 (CN)
(74) Representative: Gille Hrabal
(86) International application number: PCT/CN2015/091842
(87) International publication number: WO 2016/197497

(57) **Abstract**

The present invention relates to the technical field of antibody engineering. Provided are a full human anti-PD-1 monoclonal antibody, a preparation method therefor, and uses thereof. Anti-PD-1 monoclonal antibodies are obtained from a fully synthetic antibody library by means of screening, and then light-chain CDR1-3-region and heavy-chain CDR1-3-region mutation construction libraries of the obtained antibody are screened in sequence by means of an affinity maturation technology to obtain a high-affinity anti-PD-1 antibody. The antibody can be used for treating tumors, inflammations, and autoimmune diseases.

SEQUENCE LISTING

<110>  Beijing Jingyitaixiang Technology Development Co.Ltd.

<120>  ANTI-PD-1 ANTIBODIES AND THEREOF

<130>  160202-083WT

<160>  50

<170>  PatentIn version 3.5

<210>  1
<211>  113
<212>  PRT
<213>  Artificial Sequence

<220>
<223>  The sequence is synthesized

<400>  1

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Asn
            20                  25                  30

Gly Met His Trp Val Arg Gln Ala Pro Gly Lys Gly Leu Glu Trp Val
        35                  40                  45

Ala Val Ile Trp Tyr Asp Ser Thr Lys Lys Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Thr Asp Tyr Trp Gly Gln Gly Thr Leu Val Thr Val Ser
            100                 105                 110

Ser

<210>  2
<211>  113
<212>  PRT
<213>  Artificial Sequence

<220>
<223>  The sequence is synthesized

<400>  2

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Tyr
            20                  25                  30

Gly Met His Trp Val Arg Gln Ala Pro Gly Lys Gly Leu Glu Trp Val
        35                  40                  45

Ala Val Ile Trp Tyr Asp Ser Ser Arg Lys Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asn Asp Tyr Trp Gly Gln Gly Thr Leu Val Thr Val Ser
            100                 105                 110

Ser

<210>  3
<211>  113
<212>  PRT
<213>  Artificial Sequence

<220>
<223>  The sequence is synthesized

<400>  3

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Asn
            20                  25                  30

Gly Met His Trp Val Arg Gln Ala Pro Gly Lys Gly Leu Glu Trp Val
        35                  40                  45

Ala Val Ile Trp Tyr Asp Ser Ser Arg Lys Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asn Asp Tyr Trp Gly Gln Gly Thr Leu Val Thr Val Ser
            100                 105                 110

Ser

<210>  4
<211>  113
<212>  PRT
<213>  Artificial Sequence

<220>
<223>  The sequence is synthesized

<400>  4

Gln Val Gln Leu Val Glu Ser Gly Gly Gly Val Val Gln Pro Gly Arg
1               5                   10                  15

Ser Leu Arg Leu Asp Cys Lys Ala Ser Gly Ile Thr Phe Ser Asn Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Lys Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Ser Leu Arg Ala Glu Asp Thr Ala Val Tyr Tyr Cys
                85                  90                  95

Ala Thr Asn Asn Asp Tyr Trp Gly Gln Gly Thr Leu Val Thr Val Ser
            100                 105                 110

Ser

<210>  5
<211>  107
<212>  PRT
<213>  Artificial Sequence

<220>
<223>  The sequence is synthesized

<400>  5

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Ile His Ser Tyr
            20                  25                  30

Leu Asp Trp Tyr Gln Gln Lys Pro Gly Lys Ala Pro Lys Leu Leu Ile
        35                  40                  45

Tyr Glu Ala Ser Thr Arg Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Leu Lys Leu Pro Ile
                85                  90                  95

Thr Phe Gly Gln Gly Thr Lys Val Glu Ile Lys
            100                 105

<210>  6
<211>  107
<212>  PRT
<213>  Artificial Sequence

<220>
<223>  The sequence is synthesized

<400>  6

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Val Ser Asn Trp
            20                  25                  30

Leu Asp Trp Tyr Gln Gln Lys Pro Gly Lys Ala Pro Lys Leu Leu Ile
        35                  40                  45

Tyr Asp Ala Ser Asn Arg Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Arg His Ile Pro Leu
                85                  90                  95

Thr Phe Gly Gln Gly Thr Lys Val Glu Ile Lys
            100                 105

<210>  7
<211>  107
<212>  PRT
<213>  Artificial Sequence

<220>
<223>  The sequence is synthesized

<400>  7

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile His Asn Tyr
            20                  25                  30

Leu Asp Trp Tyr Gln Gln Lys Pro Gly Lys Ala Pro Lys Leu Leu Ile
        35                  40                  45

Tyr Asn Ala Ser Thr Arg Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Glu Leu His Leu Pro Leu
                85                  90                  95

Thr Phe Gly Gln Gly Thr Lys Val Glu Ile Lys
            100                 105

<210>  8
<211>  107
<212>  PRT
<213>  Artificial Sequence

<220>
<223>  The sequence is synthesized

<400>  8

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asn Asn Trp
            20                  25                  30

Leu Asp Trp Tyr Gln Gln Lys Pro Gly Lys Ala Pro Lys Leu Leu Ile
        35                  40                  45

Tyr Asp Ala Ser Thr Leu Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asn Val Asn Leu Pro Leu
                85                  90                  95

Thr Phe Gly Gln Gly Thr Lys Val Glu Ile Lys
            100                 105

<210>  9
<211>  107
<212>  PRT
<213>  Artificial Sequence

<220>
<223>  The sequence is synthesized

<400>  9

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Arg Asn Tyr
            20                  25                  30

Leu Asp Trp Tyr Gln Gln Lys Pro Gly Lys Ala Pro Lys Leu Leu Ile
        35                  40                  45

Tyr Gly Ala Ser Thr Arg Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Ile Asp Leu Pro Leu
                85                  90                  95

Thr Phe Gly Gln Gly Thr Lys Val Glu Ile Lys
            100                 105

<210>  10
<211>  107
<212>  PRT
<213>  Artificial Sequence

<220>
<223>  The sequence is synthesized

<400>  10

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Asn Ser Trp
            20                  25                  30

Leu Asp Trp Tyr Gln Gln Lys Pro Gly Lys Ala Pro Lys Leu Leu Ile
        35                  40                  45

Tyr Asp Ala Ser Thr Arg Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Arg Leu Pro Leu
                85                  90                  95

Thr Phe Gly Gln Gly Thr Lys Val Glu Ile Lys
            100                 105


<210>  11
<211>  107
<212>  PRT
<213>  Artificial Sequence

<220>
<223>  The sequence is synthesized

<400>  11

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Ser Asn Tyr
            20                  25                  30

Leu Asp Trp Tyr Gln Gln Lys Pro Gly Lys Ala Pro Lys Leu Leu Ile
        35                  40                  45

Tyr Asp Ala Ser Thr Arg Ala Thr Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asn Met Gln Leu Pro Leu
                85                  90                  95

Thr Phe Gly Gln Gly Thr Lys Val Glu Ile Lys
            100                 105

<210>  12
<211>  993
<212>  DNA
<213>  Artificial Sequence

<220>
<223>  The sequence is synthesized

<400>  12
gtgtcctccg cctccaccaa gggcccttcc gtgttccctc tggccccttg ctcccgctcc      60

acctccgagt ccaccgccgc cctgggctgc ctggtgaagg actacttccc tgagcctgtg     120

accgtgtcct ggaactccgg cgccctgacc tccggcgtgc acaccttccc tgccgtgctg     180

cagtcctccg gcctgtactc cctgtcctcc gtggtgaccg tgccttcctc ctccctgggc     240

accaagacct acacctgcaa cgtggaccac aagccttcca acaccaaggt ggacaagcgc     300

gtggagtcca agtacggccc tccttgccct ccttgccctg cccctgagtt cctgggcggc     360

ccttccgtgt tcctgttccc tcctaagcct aaggacaccc tgatgatctc ccgcacccct     420

gaggtgacct gcgtggtggt ggacgtgtcc caggaggacc ctgaggtgca gttcaactgg     480

tacgtggacg gcgtggaggt gcacaacgcc aagaccaagc ctcgcgagga gcagttcaac     540

tccacctacc gcgtggtgtc cgtgctgacc gtgctgcacc aggactggct gaacggcaag     600

gagtacaagt gcaaggtgtc caacaagggc ctgccttcct ccatcgagaa gaccatctcc     660

aaggccaagg gccagcctcg cgagcctcag gtgtacaccc tgcctccttc ccaggaggag     720

atgaccaaga accaggtgtc cctgacctgc ctggtgaagg gcttctaccc ttccgacatc     780

gccgtggagt gggagtccaa cggccagcct gagaacaact acaagaccac ccctcctgtg     840

ctggactccg acggctcctt cttcctgtac tcccgcctga ccgtggacaa gtcccgctgg     900

caggagggca acgtgttctc ctgctccgtg atgcacgagg ccctgcacaa ccactacacc     960

cagaagtccc tgtccctgtc cctgggcaag tag                                  993

<210>  13
<211>  107
<212>  PRT
<213>  Artificial Sequence

<220>
<223>  The sequence is synthesized

<400>  13

<210>  14
<211>  330
<212>  PRT
<213>  Artificial Sequence

<220>
<223>  The sequence is synthesized

<400>  14

Val Ser Ser Ala Ser Thr Lys Gly Pro Ser Val Phe Pro Leu Ala Pro
1               5                   10                  15

Cys Ser Arg Ser Thr Ser Glu Ser Thr Ala Ala Leu Gly Cys Leu Val
            20                  25                  30

Lys Asp Tyr Phe Pro Glu Pro Val Thr Val Ser Trp Asn Ser Gly Ala
        35                  40                  45

Leu Thr Ser Gly Val His Thr Phe Pro Ala Val Leu Gln Ser Ser Gly
    50                  55                  60

Leu Tyr Ser Leu Ser Ser Val Val Thr Val Pro Ser Ser Ser Leu Gly
65                  70                  75                  80

Thr Lys Thr Tyr Thr Cys Asn Val Asp His Lys Pro Ser Asn Thr Lys
                85                  90                  95

Val Asp Lys Arg Val Glu Ser Lys Tyr Gly Pro Pro Cys Pro Pro Cys
            100                 105                 110

Pro Ala Pro Glu Phe Leu Gly Gly Pro Ser Val Phe Leu Phe Pro Pro

115                 120                 125

Lys Pro Lys Asp Thr Leu Met Ile Ser Arg Thr Pro Glu Val Thr Cys
    130                 135                 140

Val Val Val Asp Val Ser Gln Glu Asp Pro Glu Val Gln Phe Asn Trp
145                 150                 155                 160

Tyr Val Asp Gly Val Glu Val His Asn Ala Lys Thr Lys Pro Arg Glu
                165                 170                 175

Glu Gln Phe Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Gly Leu Pro Ser Ser Ile Glu Lys Thr Ile Ser Lys Ala Lys Gly
    210                 215                 220

Gln Pro Arg Glu Pro Gln Val Tyr Thr Leu Pro Pro Ser Gln Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Arg Leu Thr Val Asp Lys Ser Arg Trp Gln Glu Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Leu Gly Lys
                325                 330

<210>  15
<211>  321
<212>  DNA
<213>  Artificial Sequence

<220>
<223>  The sequence is synthesized

<400>  15
cgtacggtgg cggcgccatc tgtcttcatc ttcccgccat ctgatgagca gttgaaatct      60

ggtaccgcta gcgttgtgtg cctgctgaat aacttctatc ccagagaggc caaagtacag     120

tggaaggtgg ataacgccct ccaatcgggt aactcccagg agagtgtcac agagcaggac     180

agcaaggaca gcacctacag cctcagcagc accctgacgc tgagcaaagc agactacgag     240

aaacacaaag tctacgcctg cgaagtcacc catcagggcc tgagctcgcc cgtcacaaag     300

agcttcaaca ggggagagtg t                                               321


<210>  16
<211>  33
<212>  DNA
<213>  Artificial Sequence

<220>
<223>  The sequence is synthesized

<400>  16
ctggtcaccg tctcctcagg tggtggtggt agc                                   33


<210>  17
<211>  32
<212>  DNA
<213>  Artificial Sequence

<220>
<223>  The sequence is synthesized

<400>  17
ctaagcggcc gctttgatct ccactttggt gc                                    32


<210>  18
<211>  11
<212>  PRT
<213>  Artificial Sequence

<220>
<223>  The sequence is synthesized

<400>  18

Arg Ala Ser Gln Asn Ile His Ser Tyr Leu Asp
1               5                   10


<210>  19
<211>  11
<212>  PRT
<213>  Artificial Sequence

<220>
<223>  The sequence is synthesized

<400>  19

Arg Ala Ser Gln Asn Val Ser Asn Trp Leu Asp

1               5                   10


<210>  20
<211>  11
<212>  PRT
<213>  Artificial Sequence

<220>
<223>  The sequence is synthesized

<400>  20

Arg Ala Ser Gln Ser Ile His Asn Tyr Leu Asp
1               5                   10


<210>  21
<211>  11
<212>  PRT
<213>  Artificial Sequence

<220>
<223>  The sequence is synthesized

<400>  21

Arg Ala Ser Gln Asp Ile Asn Asn Trp Leu Asp
1               5                   10


<210>  22
<211>  11
<212>  PRT
<213>  Artificial Sequence

<220>
<223>  The sequence is synthesized

<400>  22

Arg Ala Ser Gln Asp Val Arg Thr Tyr Leu Asp
1               5                   10


<210>  23
<211>  11
<212>  PRT
<213>  Artificial Sequence

<220>
<223>  The sequence is synthesized

<400>  23

Arg Ala Ser Gln Gly Ile Asn Ser Trp Leu Asp
1               5                   10


<210>  24
<211>  11
<212>  PRT
<213>  Artificial Sequence

<220>
<223>  The sequence is synthesized

<400>  24

Arg Ala Ser Gln Ser Val Ser Asn Tyr Leu Asp
1               5                   10


<210>  25
<211>  7
<212>  PRT
<213>  Artificial Sequence

<220>
<223>  The sequence is synthesized

<400>  25

Glu Ala Ser Thr Arg Ala Ser
1               5


<210>  26
<211>  7
<212>  PRT
<213>  Artificial Sequence

<220>
<223>  The sequence is synthesized

<400>  26

Asp Ala Ser Asn Arg Ala Thr
1               5


<210>  27
<211>  7
<212>  PRT
<213>  Artificial Sequence

<220>
<223>  The sequence is synthesized

<400>  27

Asn Ala Ser Thr Arg Ala Thr
1               5


<210>  28
<211>  7
<212>  PRT
<213>  Artificial Sequence

<220>
<223>  The sequence is synthesized

<400>  28

Asp Ala Ser Thr Leu Ala Thr

1               5


<210>  29
<211>  7
<212>  PRT
<213>  Artificial Sequence

<220>
<223>  The sequence is synthesized

<400>  29

Gly Ala Ser Thr Arg Ala Thr
1               5


<210>  30
<211>  7
<212>  PRT
<213>  Artificial Sequence

<220>
<223>  The sequence is synthesized

<400>  30

Asp Ala Ser Thr Arg Ala Thr
1               5


<210>  31
<211>  9
<212>  PRT
<213>  Artificial Sequence

<220>
<223>  The sequence is synthesized

<400>  31

Gln Gln Ala Leu Lys Leu Pro Ile Thr
1               5


<210>  32
<211>  9
<212>  PRT
<213>  Artificial Sequence

<220>
<223>  The sequence is synthesized

<400>  32

Gln Gln Ser Arg His Ile Pro Leu Thr
1               5


<210>  33
<211>  9
<212>  PRT
<213>  Artificial Sequence

<220>
<223>  The sequence is synthesized

<400>  33

Gln Gln Glu Leu His Leu Pro Leu Thr
1               5


<210>  34
<211>  9
<212>  PRT
<213>  Artificial Sequence

<220>
<223>  The sequence is synthesized

<400>  34

Gln Gln Asn Val Asn Leu Pro Leu Thr
1               5


<210>  35
<211>  9
<212>  PRT
<213>  Artificial Sequence

<220>
<223>  The sequence is synthesized

<400>  35

Gln Gln Asp Ile Asp Leu Pro Leu Thr
1               5


<210>  36
<211>  9
<212>  PRT
<213>  Artificial Sequence

<220>
<223>  The sequence is synthesized

<400>  36

Gln Gln Ser Tyr Arg Leu Pro Leu Thr
1               5


<210>  37
<211>  9
<212>  PRT
<213>  Artificial Sequence

<220>
<223>  The sequence is synthesized

<400>  37

Gln Gln Asn Met Gln Leu Pro Leu Thr

1                 5


<210>  38
<211>  6
<212>  PRT
<213>  Artificial Sequence

<220>
<223>  The sequence is synthesized

<400>  38

Ser Asn Asn Gly Met His
1               5


<210>  39
<211>  6
<212>  PRT
<213>  Artificial Sequence

<220>
<223>  The sequence is synthesized

<400>  39

Ser Asn Tyr Gly Met His
1               5


<210>  40
<211>  9
<212>  PRT
<213>  Artificial Sequence

<220>
<223>  The sequence is synthesized

<400>  40

Val Ile Trp Tyr Asp Gly Ser Lys Lys
1               5


<210>  41
<211>  9
<212>  PRT
<213>  Artificial Sequence

<220>
<223>  The sequence is synthesized

<400>  41

Val Ile Trp Tyr Asp Ser Ser Arg Lys
1               5


<210>  42
<211>  9
<212>  PRT
<213>  Artificial Sequence

<220>
<223>  The sequence is synthesized

<400>  42

Val Ile Trp Tyr Asp Ser Thr Lys Lys
1               5


<210>  43
<211>  13
<212>  PRT
<213>  Artificial Sequence

<220>
<223>  The sequence is synthesized

<400>  43

Thr Ala Val Tyr Tyr Cys Ala Thr Asn Asn Asp Tyr Trp
1               5                   10


<210>  44
<211>  13
<212>  PRT
<213>  Artificial Sequence

<220>
<223>  The sequence is synthesized

<400>  44

Thr Ala Val Tyr Tyr Cys Ala Thr Asn Thr Asp Tyr Trp
1               5                   10


<210>  45
<211>  21
<212>  DNA
<213>  Artificial Sequence

<220>
<223>  The sequence is synthesized

<400>  45
gatatccaga tgacccagag c                                                 21


<210>  46
<211>  32
<212>  DNA
<213>  Artificial Sequence

<220>
<223>  The sequence is synthesized

<400>  46
ctaagcggcc gctttgatct ccactttggt gc                                     32


<210>  47

<211>  34
<212>  DNA
<213>  Artificial Sequence

<220>
<223>  The sequence is synthesized

<400>  47
cataccatgg cccaggtgca gctggtggag tctg                                    34


<210>  48
<211>  33
<212>  DNA
<213>  Artificial Sequence

<220>
<223>  The sequence is synthesized

<400>  48
gctctgggtc atctggatat cggatccacc acc                                     33


<210>  49
<211>  34
<212>  DNA
<213>  Artificial Sequence

<220>
<223>  The sequence is synthesized

<400>  49
cataccatgg cccaggtgca gctggtggag tctg                                    34


<210>  50
<211>  27
<212>  DNA
<213>  Artificial Sequence

<220>
<223>  The sequence is synthesized

<400>  50
tgaggagacg gtgaccaggg tgccctg                                            27

## Description

### TECHNICAL FIELD OF THE INVENTION

The present invention relates to an antibody, obtaining method and application thereof. Specifically, the present invention relates to human anti-PD-1 monoclonal antibodies, polynucleotide sequences or combination, vectors, host cells and drugs, obtaining method and application thereof.

### BACKGROUNG OF THE INVENTION

Immune regulation plays an important role in the immune response process of a human body, and the activation of immunocompetent cells is critical to the regulation of the immune response. Researches have shown that the activation and proliferation of T cells are dependent on dual signaling pathways. The concept of "co-stimulatory signals" was proposed by Bretscher and Cohn in 1970 on the basis of the T-cell activation dual signaling model, that is, the activation of T cells requires not only the delivery of MHC-antigen peptide complexes by APC to antigen-specific T cells to provide first signals, but also the participation of multiple co-stimulatory molecules in providing auxiliary second signals (co-stimulatory signals); with the development of researches, co-stimulatory signals have gradually become one of hot topics in immunology; and these co-stimulatory signal molecules mainly include CD28/B7 and TNFR/TNF super-families. PD-1/PD-L1, as members of the CD28/B7 super-family, can mediate negative co-stimulatory signals. PD-1/PD-L1 signaling pathway can effectively inhibit T and B cell function and T cell proliferation, while reducing the secretion of cytokines IL-2, IL-10 and IFN-γ. It plays an important role in immune regulation, and has a major significance in the study of tumor immunity, autoimmunity, transplantation immunology, asthma, viral infections and other diseases.

PD-1 belongs to immunoglobulin super-family type I transmembrane proteins, and has a molecular weight of about 50-55 KD. It was originally obtained from apoptotic T cell hybridomas by subtractive hybridization techniques, and termed as Programmed Cell Death 1, which is associated with apoptosis. The coding gene for PD-1 is PD-CD1, which is located on human chromosome 2q37.3 and has 23% homology with CTLA4 gene. PD-1 consists of intracellular, transmembrane and extracellular regions, in which the extracellular region contains an immunoglobulin variable region IgV-like domain; the two tyrosine residues at the N-terminal of the intracellular region together with other amino acid residues form an immunoreceptor tyrosine inhibitory motif (ITIM), which plays a role of antagonizing antigen receptor stimulatory signals by tyrosine phosphorylation, thereby providing the negative regulation function in the immune response process; PD-1 molecules can be inducibly expressed on the surface of activated T cells, B cells, NK cells, monocytes and dendritic cells, and combine with their ligands PD-L1 and PD-L2 to inhibit the activation of lymphocytes, thereby inhibiting the immune response of immune cells.

PD-L1 (CD274 or B7H1) and PD-L2 (CD273 or B7DC) are two ligands of PD1, and genes of these two ligands both are located on human chromosome 9p24.2, with origin in the same direction, and about 42kb interval; they belong to the B7 family, and, like other members, PD-L1 and PD-L2 consist of an IgV-like domain, an IgC-like domain, a transmembrane region and a short and conserved cytoplasmic tail; and compared with PD-L2, the cytoplasmic tail of PD-L1 is more conserved among different species. PD-L1 is inducibly expressed in activated T cells, B cells, dendritic cells, monocytes and various types of tumor cells (such as lung cancer, liver cancer, breast cancer, ovarian cancer, kidney cancer, head and neck cancer, esophageal cancer, skin cancer and squamous cell carcinoma); while PD-L2 is mainly expressed in activated macrophages, dendritic cells and individual tumor cells (such as Hodgkin's lymphoma). PD-L1 and PD-1 interact on tumor surface, leading to the apoptosis of tumor antigen-specific T cells, resulting in tumor cell escape of immune surveillance. Anti-PD-1 monoclonal antibodies promote the proliferation of tumor antigen-specific T cells by blocking the PD-1/PD-L1 signaling pathway, to play a role of killing tumor cells, thereby effectively improving the immunotherapy effect, and showing the potential to treat various types of tumors.

Currently, many large international pharmaceutical companies have been studying on monoclonal antibody drugs of PD-1 or PD-L1, in which PD-1 inhibitor Opdivo (Nivolumab), provided by Bristol-Myers Squibb Company, was approved in Japan in July, 2014; Merck@ PD-1 inhibitor was approved by FDA in September, 2014. The first indication of these two drugs is melanoma. With the advance of each company's clinical programs, indications will expand to lung cancer, breast cancer, cancer of the blood and so on.

**Table 1. Anti-PD-1 antibodies currently undergoing clinical trials**

| Name of Antibody | Development Company | Clinical Research | |
|---|---|---|---|
| | | Indication | Stage |
| nivolumab | Bristol-Myers Squibb (Princeton NJ USA) | Hodgkin's lymphoma | I |
| | | Tumor | II |
| | | Renal cell carcinoma (RCC) | II |
| | | Melanoma | Listed |
| | Ono Pharmaceutical Co., Ltd (Osaka Japan) | Hepatitis C | I |
| pembrolizumab | Merck & Co., Inc. (Whitehouse Station NJ USA) | Bladder cancer | III |
| | | Non-small cell lung cancer (NSCLC) | III |
| | | Solid tumor | I |
| | | Head and neck cancer | II |
| | | Hodgkin's lymphoma | I |
| | | Melanoma | Listed |
| pidilizumab | CureTech (Yavne Israel) | Follicular lymphoma | II |
| | | Metastatic colorectal cancer | II |
| | | Pancreatic cancer | II |
| | | Hodgkin's lymphoma | II |
| | | Prostate cancer | II |
| | | Acute myeloid leukemia (AML) | II |
| | | Liver cancer | I/II |
| | | Hepatitis C | I/II |
| | | Multiple myeloma | II |

### SUMMARY OF THE INVENTION

The present invention aims at providing human anti-PD-1 monoclonal antibodies, obtaining method and application thereof.

A human anti-PD-1 monoclonal antibody DFPD1-1 is first selected from the synthetic antibody library. The DFPD1-1 was analyzed and then small-capacity mutant library was designed by computer-aided design based on this antibody. Then a mutant library of the light chain CDR1, CDR2 and CDR3 was created, higher affinity of monoclonal antibodies, DFPD1-3 and DFPD1-7, were selected by screening. A mutant library of its heavy chain CDR1, CDR 2 and CDR 3 was created based on DFPD1-1, DFPD1-3 and PFPD1-7, higher affinity anti-PD-1 monoclonal antibodies were selected.

In order to achieve the above purpose, the present invention provides an obtaining method of anti-PD-1 monoclonal antibodies, which includes:
(1): The biopanning of anti-PD-1 single-chain antibody. A high affinity antibody DFPD1-1 was obtained from a fully-synthetic ScFv phage library through three rounds of enriching and screening. Its heavy chain is DFPD1- H1 (SEQ ID NO. 1), and its light chain is DFPD1-L1 (SEQ ID NO. 5).
(2): Based on DFPD1-1, a mutant library of light chain CDR1, CDR2 and CDR3 was designed by analyzing DFPD1-1 tertiary structure with computer-aided design. A mutant library of light chain CDR1, CDR2 and CDR3 were created, bio-panned and screened. By identifying the positive clones and comparing the affinity of single-chain antibodies on the phage level, six different antibody light chains (DFPD1-2, DFPD1-3, DFPD1-4, DFPD1-5, DFPD1-6 and DFPD1-7) were obtained, and their corresponding light chain sequences were DFPD1-L2 (SEQ ID NO. 6), DFPD1-L3 (SEQ ID NO. 7), DFPD1-L4 (SEQ ID NO. 8), DFPD1-L5 (SEQ ID NO. 9), DFPD1-L6 (SEQ ID NO. 10), DFPD1-L7 (SEQ ID NO. 11).
(3):Based on two higher affinity clones DFPD1-3 and DFPD1-7, a mutant library of heavy chain CDR1,CDR2 and CDR3 was designed, bio-panned and screened, five different single-chain antibodies (clone No: DFPD1-9, DFPD1-10, DFPD1-11, DFPD1-12 and DFPD1-13) were selected. Wherein, the light chain variable region sequence of DFPD1-9, DFPD1-11 and DFPD1-12 is DFPD1-L3, and the light chain variable region sequence of DFPD1-10 and DFPD1-13 is DFPD1-L7. The heavy chain variable region sequence of DFPD1-9 and DFPD1-10 is DFPD1-H2 (SEQ ID NO. 2), the heavy chain variable region sequence of DFPD1-11 and DFPD1-13 is DFPD1-H3 (SEQ ID NO. 3), the heavy chain variable region sequence of DFPD1-12 is DFPD1-H4 (SEQ ID NO. 4).
(4): Genes which encode the variable region of the heavy chain and the light chain of the monoclonal antibodies were described in step 3 . The corresponding constant region genes were cloned into the eukaryotic expression vector and transfected into host cells. The monoclonal antibodies were purified, then affinity and other biological functions of whole monoclonal antibodies were compared.

The anti-PD-1 monoclonal antibodies were obtained by the above method , including the light chain and the heavy chain, the light chain CDR1, CDR2 and CDR3 are represented by LCDR1, LCDR2 and LCDR3, LCDR1 is preferably RASQNIHSYLD (SEQ ID NO.18), RASQNVSNWLD(SEQ ID NO.19), RASQSIHNYLD(SEQ ID NO.20), RASQDINNWLD(SEQ ID NO.21), RASQDVRTYLD(SEQ ID NO.22), RASQGINSWLD(SEQ ID NO.23) or RASQSVSNYLD(SEQ ID NO.24), LCDR2 is preferably EASTRAS(SEQ ID NO.25), DASNRAT (SEQ ID NO.26), NASTRAT(SEQ ID NO.27), DASTLAT(SEQ ID NO.28), GASTRAT(SEQ ID NO.29) or DASTRAT(SEQ ID NO.30), LCDR3 is preferably QQALKLPIT(SEQ ID NO.31), QQSRHIPLT(SEQ ID NO.32), QQELHLPLT(SEQ ID NO.33), QQNVNLPLT(SEQ ID NO.34), QQDIDLPLT(SEQ ID NO.35), QQSYRLPLT(SEQ ID NO.36) or QQNMQLPLT(SEQ ID NO.37).

Wherein, the amino acid sequence of the light chain variable region is preferably SEQ ID NO. 5, SEQ ID NO. 6, SEQ ID NO. 7, SEQ ID NO. 8, SEQ ID NO. 9, SEQ ID NO. 10 or SEQ ID NO. 11.

The anti-PD-1 monoclonal antibodies were obtained by the above method, including the light chain and heavy chain, the heavy chain CDR1, CDR2 and CDR3 is represented by HCDR1, HCDR2 and HCDR3, HCDR1 is preferably SNNGMH(SEQ ID NO.38) or SNYGMH(SEQ ID NO.39), HCDR2 is preferably VIWYDGSKK(SEQ ID NO.40), VIWYDSSRK(SEQ ID NO.41) or VIWYDSTKK(SEQ ID NO.42), HCDR3 is preferably TAVYYCATNNDYW (SEQ ID NO.43) or TAVYYCATNTDYW(SEQ ID NO.44).

Wherein, the amino acid sequence of the heavy chain variable region is preferably SEQ ID NO. 1, SEQ ID NO. 2, SEQ ID NO. 3 or SEQ ID NO. 4.

This invention also provides antibodies, polypeptides or proteins which contain said light chain or heavy chain.

This invention also provides antibodies that contain said light chain or heavy chain. And said antibodies can block the binding of PD-1to its ligand PD-L1, consequently inhibit the biological activity of PD-1.

This invention also provides polynucleotide sequences or combinations which encode said light chain or heavy chain.

This invention also provides recombinant DNA expression vectors which contain DNA sequences encoding the variable regions and/or the constant regions of the heavy chain and the light chain of the anti-PD-1 antibody.

This invention also provides host cells transfected with the said recombinant DNA expression vectors, while the host cells are preferably *E. coli* and other prokaryotic cells, yeasts, insects or mammalian cells.

Preferably, the host cells are HEK293E cells, CHO cells or NS0 cells and so on.

This invention also provides whole antibodies, single domain antibodies, bi-specific antibodies, antibody-drug conjugates and/or chimeric antigen receptor T-cell immunotherapy which contain said sequences.

This invention also provides monoclonal antibodies, artificial vectors, a drug or combinations of drugs which contain the said light chain or heavy chain.

This invention also provides detection reagents or kits which contain the said light chain or heavy chain.

Wherein, the anti-PD-1 monoclonal antibody contains whole antibody and its fragments, the fragments include, but not limited to Fab, Fab', F (ab') 2, Fv or ScFv.

Wherein, the full length antibodies are human monoclonal antibodies.

Wherein, the constant region of the heavy chain of anti-PD-1 monoclonal antibody is IgG1, IgG2, IgG3 or IgG4, the constant region of the light chain is Cκ or C_{λ}.

Preferably, the constant region of the heavy chain is IgG4.

Preferably, the constant region of the light chain is Cκ.

The CDR is the abbreviation of complementary determining region, the ScFv is the abbreviation of single-chain fragment variable, the CAR-T is the abbreviation of chimeric antigen receptor T-cell immunotherapy, the Fab is the abbreviation of antigen binding fragment, the HEK293E cell is human embryonic kidney 293E cell, the CHO cell is china hamster ovary cell, the NS0 cell is NS0 mouse thymoma cell.

Compared with the prior art, the beneficial effects of the present invention can prevent and treat diseases by inhibiting the activity of PD-1 , wherein the diseases are selected from the group consisting of cancer, infectious diseases or immune system disorders. Types of cancer include, but are not limited to lung cancer, kidney cancer, melanoma, breast cancer, liver cancer, head and neck cancer, skin cancer, squamous cell carcinoma, ovarian cancer, bone cancer, colorectal cancer, bladder cancer, stomach cancer, pancreatic cancer, prostate cancer, Hodgkin's lymphoma, follicular lymphoma, chronic or acute leukemia or solid tumors. Infectious diseases include, but not limited to HIV infection, hepatitis virus (type A, B and C) infection, herpes virus infection or influenza virus infection. Immune system disorders include, but not limited to lupus erythematosus, rheumatoid arthritis, ankylosing spondylitis, myasthenia gravis, multiple sclerosis, autoimmune hemolytic anemia, autoimmune hepatitis, scleroderma, poly-arteritis or Wegener's granulomatosis.

### BRIEF DESCRIPTION OF THE DRAWINGS

Fig.1 shows the plasmid atlas of pScFvDisb-s.
Fig.2 shows the electrophoresis atlas of the PCR product of the heavy chain and the linker region by using DFPD1-1 as the template in building the mutant light chain variable region library.
Fig.3 shows the electrophoresis atlas of the PCR product obtained by using the synthetic mutant light chain library as template in building the mutant light chain variable region library.
Fig.4 shows the electrophoresis atlas of the PCR product in building the mutant light chain variable region library VLCDR123M-DFPD1-1.
Fig.5 shows the electrophoresis atlas of the double digested product of plasmid pScFvDisb-s in building the mutant light chain variable region library. and heavy chain variable region library by NcoI-HF and NotI.
Fig.6 shows relative affinity identification of the phage-Abs selected from the mutant light chain variable region library by monoclonal phage-ELISA.
Fig.7 shows relative affinity identification of the phage-Abs selected from the mutant light chain variable region library by gradient diluting phage-ELISA
Fig.8 shows that the electrophoresis atlas of the PCR product by using the synthetic mutant heavy chain variable domain library as template in building the mutant heavy chain variable region library
Fig.9 shows that the electrophoresis atlas of the PCR product of the light chain and the linker by using the plasmid DFPD1-3 and DFPD1-7 as template in building the mutant heavy chain variable region library
Fig.10 shows the electrophoresis atlas of the PCR product in building the mutant heavy chain variable region library VHCDR123M-DFPD1-3
Fig.11 shows the electrophoresis atlas of the PCR product of the mutant library obtained by amplification in building the mutant heavy chain variable region library VHCDR123M-DFPD1-7.
Fig.12 shows relative affinity identification of the phage-Abs selected from the mutant heavy chain variable region library by monoclonal phage-ELISA.
Fig.13 shows the relative affinity identification of phage-Abs selected from the mutant heavy chain variable region library by gradient diluting phage-ELISA Fig.14 shows the plasmid vector map of pTSE
Fig. 15 shows the binding activities of monoclonal antibodies to PD-1 at the protein level.
Fig.16 shows the competitive inhibition of PD-1 binding to PD-L1 by full antibodies.
Fig. 17 shows the binding activities of monoclonal antibodies to PD-1 on the cell surface.

### DETAILED DESCRIPTION OF EXEMPLARY EMBODIMENTS

The embodiment mode of this invention is described in the following examples. However, it should be noted that the embodiment is not limited to certain details of these examples.

The experimental methods described in the following examples are all common technologies unless otherwise specified; the reagents and biological described are all commercially available unless otherwise specified.

The present invention provides a monoclonal antibody, which specifically interacts with PD-1, the heavy chain variable region sequence is selected from the group consisting of SEQ ID NO. 1, SEQ ID NO. 2, SEQ ID NO. 3 or SEQ ID NO. 4, the light chain variable region sequence is selected from the group consisting of SEQ ID NO. 5 , SEQ ID NO. 6, SEQ ID NO. 7, SEQ ID NO. 8, SEQ ID NO. 9, SEQ ID NO. 10 or SEQ ID NO. 11.

Preferably, the heavy chain variable region sequence is SEQ ID NO. 2, SEQ ID NO. 3 or SEQ ID NO. 4, the light chain variable region sequence is SEQ ID NO. 7 or SEQ ID NO. 11.

Through screening the phage library of the light chain, the amino acid sequence of the LCDR1, LCDR2 or LCDR3 of the light chain or its functional fragment of the monoclonal antibody are selected from the following group (as shown in Table 1).

**TABLE 1**

| The Amino Acid Sequence Of Each CDR In The Light Chain | | | |
|---|---|---|---|
| No. | LCDR1 | LCDR2 | LCDR3 |
| A | RASQNIHSYLD | EASTRAS | QQALKLPIT |
| | (SEQ ID NO. 18) | (SEQ ID NO. 25) | (SEQ ID NO. 31) |
| B | RASQNVSNWLD | DASNRAT | QQSRHIPLT |
| | (SEQ ID NO. 19) | (SEQ ID NO. 26) | (SEQ ID NO. 32) |
| C | RASQSIHNYLD | NASTRAT | QQELHLPLT |
| | (SEQ ID NO. 20) | (SEQ ID NO. 27) | (SEQ ID NO. 33) |
| D | RASQDINNWLD | DASTLAT | QQNVNLPLT |
| | (SEQ ID NO. 21) | (SEQ ID NO. 28) | (SEQ ID NO. 34) |
| E | RASQDVRTYLD | GASTRAT | QQDIDLPLT |
| | (SEQ ID NO. 22) | (SEQ ID NO. 29) | (SEQ ID NO. 35) |
| F | RASQGINSWLD | DASTRAT | QQSYRLPLT |
| | (SEQ ID NO. 23) | (SEQ ID NO. 30) | (SEQ ID NO. 36) |
| G | RASQSVSNYLD | DASTRAT | QQNMQLPLT |
| | (SEQ ID NO. 24) | (SEQ ID NO. 30) | (SEQ ID NO. 37) |

Through screening the phage library of the heavy chain, the CDR1, CDR2 or CDR3 of the heavy chain or its functional fragment of the monoclonal antibodies are represented by HCDR1, HCDR2 or HCDR3, HCDR1 contains SNNGMH (SEQ ID NO. 38) or SNYGMH (SEQ ID NO.39), HCDR2 contains VIWYDGSKK (SEQ ID NO. 40), VIWYDSSRK (SEQ ID NO. 41) or VIWYDSTKK (SEQ ID NO. 42), HCDR3 contains TAVYYCATNNDYW (SEQ ID NO. 43) or TAVYYCATNTDYW (SEQ ID NO. 44).

Preferably, through screening the phage library of the heavy chain, the heavy chain variable region of the monoclonal antibody specifically interacted with PD-1 contains HCDR1, HCDR2 and HCDR3 sequence, and the light chain variable region contains LCDR1, LCDR2 and LCDR3 sequence. Wherein, HCDR1 sequence of the heavy chain variable region is selected from the amino acid sequence of SNNGMH(SEQ ID NO. 38) or SNYGMH(SEQ ID NO. 39), LCDR1 sequence of the light chain variable region is selected from the amino acid sequence of RASQSIHNYLD (SEQ ID NO. 20) or RASQSVSNYLD(SEQ ID NO. 24), HCDR2 sequence of the heavy chain variable region is selected from the amino acid sequence of VIWYDGSKK (SEQ ID NO. 40) or VIWYDSSRK (SEQ ID NO. 41), LCDR2 sequence of the light chain variable region is selected from the amino acid sequence of NASTRAT (SEQ ID NO.27) or DASTRAT (SEQ ID NO. 30), HCDR3 sequence of the heavy chain variable region is selected from the amino acid sequence of TAVYYCATNNDYW (SEQ ID NO. 43) or TAVYYCATNTDYW (SEQ ID NO. 44), LCDR3 sequence of the light chain variable region is selected from the amino acid sequence of QQELHLPLT (SEQ ID NO. 33) or QQNMQLPLT(SEQ ID NO. 37).

In the present invention, the antibody, which specifically interacts with PD-1, is obtained from the synthetic ScFv phage library, the process for preparing the anti-PD-1 monoclonal antibodies including:
First of all, anti-PD-1 single-chain antibody library was bio-panned through three rounds of enriching and screening of the antibody library, and a high affinity antibody DFPD1-1 was obtained
Secondly, a light chain CDR1, CDR2 or CDR3 mutant library was designed by computer aided design based on DFPD1-1. Six different light chain antibodies (DFPD1-2, DFPD1-3, DFPD1-4, DFPD1-5, DFPD1-6 or DFPD1-7)were identified as positive clones by bio-panning and comparing the affinities of the ScFvs on the level of phage.
Thirdly, a heavy chain CDR1, CDR2and CDR3 mutant library was built on basis of two higher affinity strains of clones DFPD1-3 and DFPD1-7. Five different single-chain antibodies which are DFPD1-9, DFPD1-10, DFPD1-11, DFPD1-12, DFPD1-13 were selected by bio-panning and comparing affinities of the ScFvs on the level of phage.

Finally, the variable region genes of the heavy chain and the light chain of the monoclonal antibody which are described above and their corresponding constant region genes were cloned into the eukaryotic expression vector and transfected into the host cells, obtained the monoclonal antibody, and then compared their affinity and other biological functions.

### EXAMPLE 1

### The Biopanning of Anti-PD-1 Single-Chain Antibody library

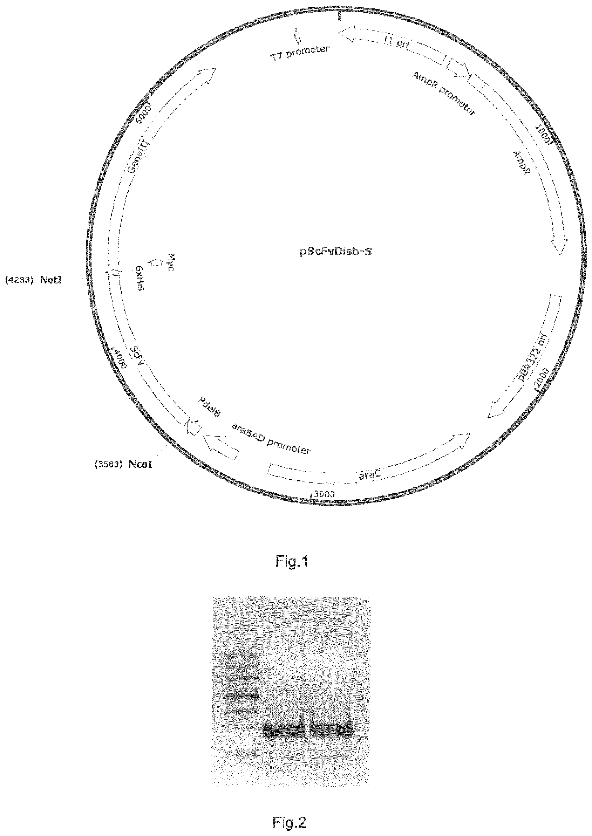

pComb3 vector (Purchased from Biovector Science Lab, Inc.) was modified by a series of cloning technology for constructing and expressing of a single-chain antibody phage library. The modified vector is named as pScFvDisb-s shown in Figure 1 and was used to make a fully-synthetic phage antibody library.

The immune tubes were coated with the antigen PD-1-His, the amount of antigen-coated is 5µg / 500 µl / tube at 4 °C overnight. The 4% skim milk / PBST was used to block the immune tubes and the full synthetic phage antibody library at room temperature for one hour. The blocked phage antibody library was added into the immune tubes for Ab-Ag interactions at room temperature for one hour, the amount of phage inputs was about 10⁹- 10¹². PBST-PBS was used to wash the unbound phages, 0.1 M Glycine-HCl (pH 2.2) was used to elute, 1.5M Tris-HCl (pH8.8) was used to neutralize the eluted phage antibody solution to about pH7.0.

The above neutralized phages infected 10ml TG1 bacteria were grown to the logarithmic period, and set for 30 minutes at in 37 °C incubator. A partial of the bacteria culture was used for gradient diluting, coated on a 2YTAG plate to calculate the amount of phage outputs. The remaining bacteria culture was centrifuged, then the supernatant was discarded. The thallus precipitation was suspended in a few of liquid culture media which was used to coat on a large 2YTAG plate for the next round of screening.

The thallus was scraped from the large plate, inoculated to 2YTAG liquid culture media, adding M13K07 for the helper phage super-infection after shaking to logarithmic period, culturing at 28 °C overnight to amplify the phages, PEG/ NaCl is used to settle and purify the phage for the next round of screening. Three rounds of enrichment and screening the phage library are carried out in total.

### EXAMPLE 2

### The Screening Of Positive Clones For The Anti-PD-1 Phage Single-Chain Antibody

After three rounds of screening, the monoclonal bacterial colonies were selected to inoculate in a 96-well deep-well plates contained 2YTAG liquid culture medium, and were cultured at 37 °C at 220 rpm to logarithmic growth period, then about 10¹⁰ helper phage M13K07 were added into each well for infection for 30 minutes at 37 °C. Then the culture was centrifuged at 4000 rpm for 15 minutes, the supernatant was discarded, the thallus was suspended with 2YTAKA. After culturing at 220 rpm 28°C overnight, the culture was centrifuged at 4000 rpm for 15 minutes at 4 °C, the phage supernatant was taken out for ELISA. The higher affinity single-chain antibody, DFPD1-1, was obtained by screening, the heavy chain variable region was named as DFPD1-H1 that has an amino acid sequence as shown in SEQ ID NO. 1, the light chain variable region was named DFPD1 - L1 that has an amino acid sequence as shown in SEQ ID NO. 5.

### EXAMPLE 3

### The Affinity Maturation Test In Vitro Of The Anti-PD-1 Single-Chain Antibody DFPD1-1

### 1. The Construction of The Mutant Library for DFPD1-1 Light Chain CDR1, CDR2 and CDR3

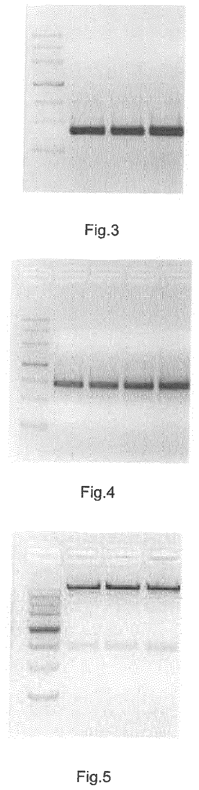

The primers PVLF1 and PVLR1were designed, using a DNA having a nucleic acid sequence shown as SEQ ID NO. 12 as a template, the light chain gene library (as shown in figure 3) were amplified by PCR; the primers PVHF1 and PVHR1, plasmid DFPD1-1 as the template were used to amplify its heavy chain and its linker (as shown in figure 2). Reaction condition: 95 °C for 30 seconds, 1 cycle, 95 °C for 15 seconds, 60 °C for 10 seconds, 72 °C for 30 seconds, 3 cycles, 95 °C for 15 second, 72 °C for 40 seconds, 25 cycles, 72 °C for 5 minutes, storing at 4 °C. The PCR products were recovered with a universal recovery kit.

The sequences of primers are as following:
PVLF1: 5'-GATATCCAGATGACCCAGAGC -3'(SEQ ID NO. 45)
PVLR1: 5'- CTAAGCGGCCGCTTTGATCTCCACTTTGGTGC-3' (SEQ ID NO. 46)
PVHF1: 5'-CATACCATGGCCCAGGTGCAGCTGGTGGAGTCTG-3' (SEQ ID NO. 47)
PVHR1: 5'-GCTCTGGGTCATCTGGATATCGGATCCACCACC-3' (SEQ ID NO. 48)

The light chain mutation variable region library of DFPD1-1 was obtained by overlap PCR via amplifying two PCR products mentioned above. Reaction condition: 95 °C for 30 seconds, 1 cycle, 95 °C for 15 seconds, 72 °C for 30 seconds, 4 cycle (added the primers PVHF1 and PVLR1), 95 °C for 15 seconds, 72 °C for 40 seconds, 25 cycles, 72 °C for 5 minutes, store at 4 °C .The PCR products were recovered with universal recovery kit, the corresponding PCR product is named as VLCDR123M-DFPD1-1 (as shown in figure 4).

The plasmid pScFvDisb-s and VLCDR123M-DFPD1-1 were digested with Ncol-HF and Notl, and the enzyme-digested products were run on 0.8% agarose gel electrophoresis (as shown in figure 5). After gel extraction, DNA was purified using a commercial available DNA purification kit. The purified digested VLCDR123M-DFPD1-1 and pScFvDisb-s was ligated at a molar ratio of 4:1 with T4 DNA Ligase for 4 hours at 16 °C. The ligation product was transformed into TG1 competent cells by the electroporation. After recovering cells for one hour at 37 °C in SOC medium, a partial of the bacteria was plated on a culture dish to estimate the capacity of library. The remaining bacteria culture was centrifuged at room temperature at 4000 rpm for 15 minutes and the supernatant was removed. The precipitation was plated on 2 YTAG large plate, and cultured at 37 °C overnight.

The capacity of the antibody library is about 10⁸. Twenty clones were picked from the antibody library randomly for sequencing, the sequences showed 95% accuracy, and the capacity of the antibody library is of high diversity.

### 2. The Bio-panning Of The Phage Antibody Library And Screening Of Positive Clones

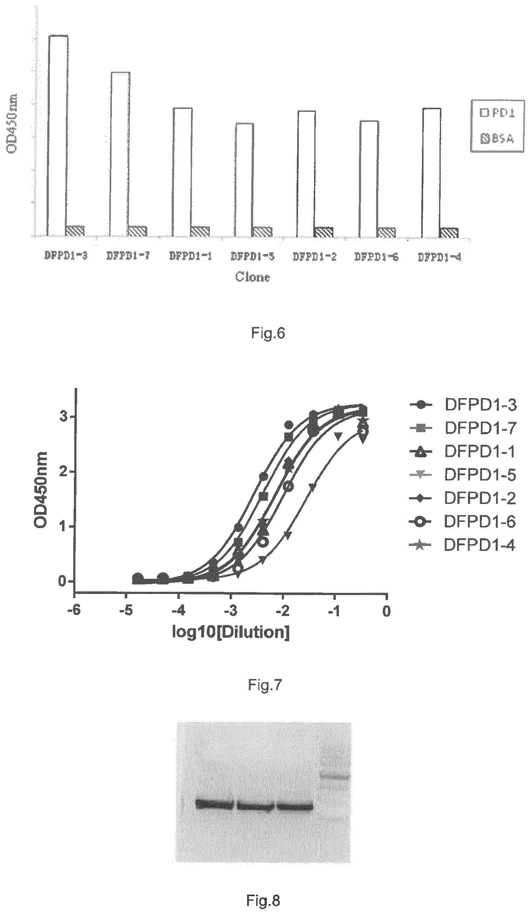

The screening is in accordance with the method of the example 1, all clones which have high affinity were sequenced, then six different clones were obtained and named DFPD1-2, DFPD1-3, DFPD1-4, DFPD1-5, DFPD1-6 and DFPD1-7, respectively, the corresponding light chain variable region is named DFPD1-L2, DFPD1-L3, DFPD1-L4, DFPD1-L5, DFPD1-L6 and DFPD1-L7, the corresponding amino acid sequence is as shown in SEQ ID NO. 6, SEQ ID NO. 7, SEQ ID NO. 8, SEQ ID NO. 9, SEQ ID NO. 10 and SEQ ID NO.11, respectively. The relative affinity of the monoclonal phage was determined with ELISA as shown in figure 6.

### 3. Determining the affinity of the anti-PD-1 antibody's ScFv by gradient diluting phage-ELISA

Displaying and purifying the phage of clones obtained by example 2, the affinity of the phage-Abs was determined by a gradient diluting phage-ELISA test.

The PD1-His in pH9.6 carbonate buffer solution was used for coating at 4 °C overnight. PBST was used for washing for three times, 4% skim milk-PBST was used for blocking at 37 °C for one hour. The purified phages were diluted for three times with 4% milk-PBST, then 100 µL diluted sample was added into each well. After setting at room temperature for one hour, the ELISA plate was washed with PBST, then the anti-M13-HRP monoclonal antibody diluted in 4% skim milk was added into the ELISA plate. After placing for one hour at room temperature, the wells were stained with TMB stain solution kit for five minutes at room temperature. The reaction was stopped with 50 µL of 2 mol/L H₂SO₄ per well, and the optical density was determined with the microplate reader by reading at 450 nm and 630 nm wavelength. The result shows a number of different phage antibodies selected can be combined with PD-1, further the affinity of DFPD1-3 and DFPD1-7 are significantly higher than other clones (as shown in figure 7). DFPD1-3 and DFPD1-7 were selected for further experiments.

### EXAMPLE 4

### The Affinity Maturation Test In Vitro of the Anti-PD-1 of Single-Chain Antibody DFPD1-3 and DFPD1-7

### 1. The construction of The Heavy Chain CDR1, CDR 2 And CDR 3 Mutant Library for DFPD1-3And DFPD1-7.

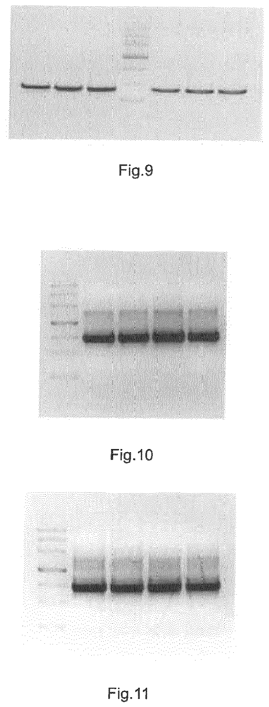

Using the synthesized heavy chain mutant library (as shown in SEQ ID NO. 13) as a template and PVHF2 and PVHR2 as PCR primers, the heavy chain gene library (as shown in figure 8) were amplified by PCR; The PVLF2 and PVLR2 as PCR primers and plasmid DFPD1-3 and DFPD1-7 were used as the template to amplify its light chain and its linker (as shown in figure 9). Wherein, the left is DFPD1-3, the right is DFPD1-7. Reaction conditions: 95 °C for 30 seconds, 1 cycle, 95 °C for 15 seconds, 60 °C for 10 seconds, 72 °C for 30 seconds, 3 cycles, 95 °C for 15 seconds, 72 °C for 40 seconds, 25 cycles, 72 °C for 5 minutes, 4 °C for storing. PCR products were recovered with universal recovery kit.

The sequences of primers as following:
PVHF2: '5CATACCATGGCCCAGGTGCAGCTGGTGGAGTCTG3' (SEQ ID NO. 49)
PVHR2: '5TGAGGAGACGGTGACCAGGGTGCCCTG 3'(SEQ ID NO. 50)
PVLF2: '5 CTGGTCACCGTCTCCTCAGGTGGTGGTGGTAGC3' (SEQ ID NO. 51)
PVLR2: '5 CTAAGCGGCCGCTTTGATCTCCACTTTGGTGC3' (SEQ ID NO. 52)

The above two PCR products were amplified by overlap PCR to obtain the gene of DFPD1-3 and DFPD1-7 heavy chain mutation library. Reaction conditions: 95 °C for 30 seconds, 1 cycle, 95 °C for 15 seconds, 72 °C for 30 seconds, 4 cycle (added the primers PVHF2 and PVLR2), 95 °C for 15 seconds, 72 °C for 40 seconds, 25 cycles, 72 °C for 5 minutes, 4 °C for storing. PCR products were recovered with universal recovery kit, the corresponding products named as VHCDR123M-DFPD1-3 (as shown in figure 10) and VHCDR123M-DFPD1-7 (as shown in figure 11).

VHCDR123M-DFPD1-3, VHCDR123M-DFPD1-7 and plasmid pScFvDisb-s were digested with NcoI-HF and NotI, and the enzyme-digested products were separated by 0.8% agarose gel electrophoresis (as shown in figure 5). After gel extraction, the enzyme-digested products were purified with a commercial available DNA purification kit. The digested PCR products and pScFvDisb-s were ligated at the molar ratio of 4:1 with T4 DNA ligase for 16 °C for 4 hours. The ligated products were transformed into TG1 competent cells by the electroporation. After recovering cells in SOC medium at 37 °C for one hour, a small fraction of the bacteria was used to plate on a culture dish to estimate the capacity of the antibody library. The remaining bacteria were centrifuged at room temperature at 4000 rpm for 15 minutes, and then the supernatant was removed. The precipitation was plated on 2 YTAG large culture dish that was culturing at 37 °C overnight.

Two different antibody libraries were made; the capacity of each antibody library is about 10⁷, the capacity of the antibody library is much higher than the diversity. Twenty clones from the above antibody library randomly were sequenced, the sequences showed 90% accuracy.

### 2. Biopanning Of The Phage Antibody Library And The Screening Of Positive Clones

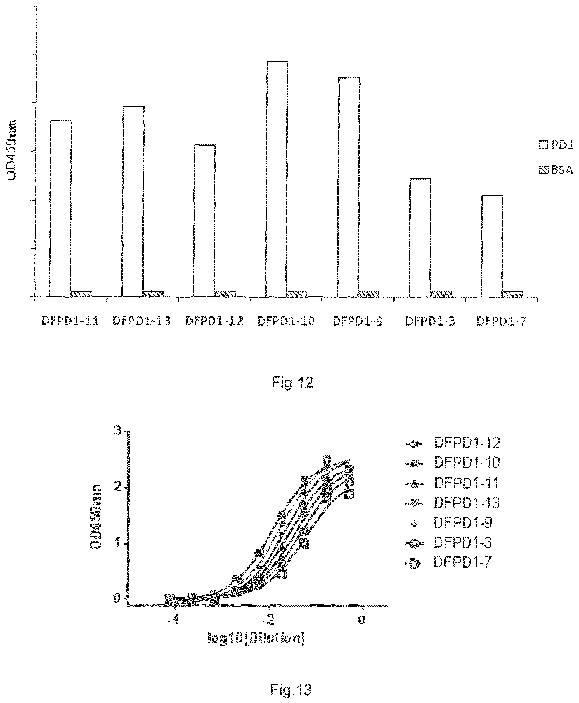

The above two antibody libraries were displayed, precipitated and purified on the phage level. Then the anti-PD-1 ScFv form antibodies were bio-panned from these libraries .The method of biopanning the phage antibody libraries is the same as example 1. The method of screening the positive clones of the anti-PD-1 antibody's ScFv is the same as example 2. The result shows five different anti-PD-1 antibodies were screened. They are named as DFPD1-9, DFPD1-10, DFPD1-11, DFPD1-12, DFPD1-13. Wherein the light chain variable region sequence of DFPD1-9, DFPD1-11 and DFPD1-12 is DFPD1-L3; and the light chain variable region sequence of DFPD1-10 and DFPD1-13 is DFPD1-L7; and the heavy chain variable region sequence of DFPD1-9 and DFPD1- 10 is DFPD1-H2; the heavy chain variable region sequence of DFPD1-11 and DFPD1-13 is DFPD1-H3; the heavy chain variable region sequence of DFPD1-12 is DFPD1-H4. The relative affinity of the monoclonal phage was determined by ELISA as shown in figure 12.

### 3. Determining the affinity of the anti-PD-1 antibody's ScFv by a gradient diluting phage-ELISA

Displaying and purifying the clones were done as descripted in the second implementation of this example at the level of monoclonal phage; the affinity of the phage-Abs was determined by a gradient diluting phage-ELISA test, and the method is the same as the third implementation of example 3. The result shows a number of different phage antibodies can interact with PD-1. There is no obvious affinity difference among this phage antibodies (as shown in figure 13), wherein DFPD1-9, DFPD1-10, DFPD1-11, DFPD1-12 and DFPD1-13 are better, and are used for the further experiments.

### EXAMPLE 5

### The Determination of the Affinity of the Anti-PD-1 Monoclonal Antibodies, DFPD1-9, DFPD1-10, DFPD1-11, DFPD1-12 and DFPD1-13

### 1. The Preparing of Anti-PD-1 Full-length Antibody

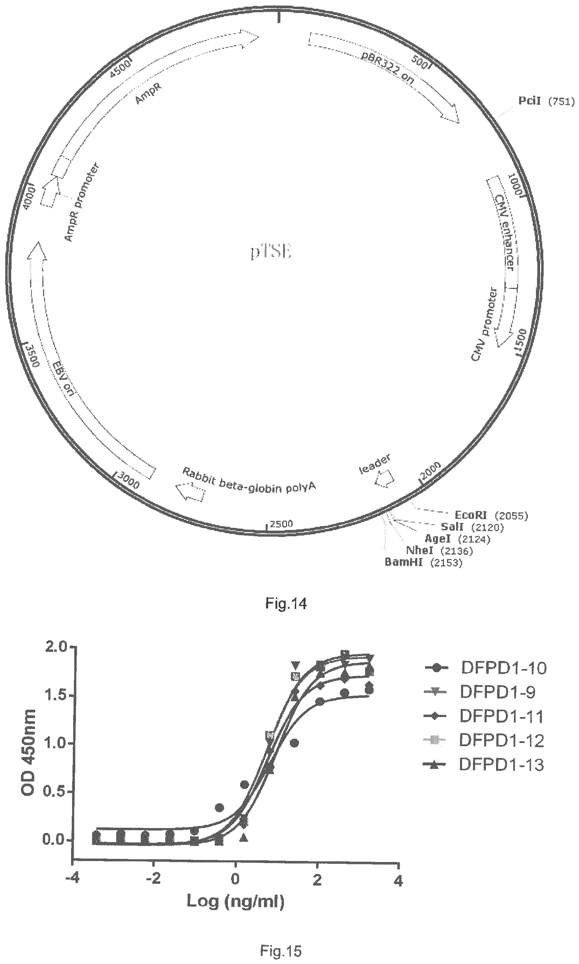

The DNAs encoding above antibodies' heavy chain VH and light chain VK were cloned into vector pTSE, respectively, with the DNAs encoding the heavy chain constant region, the light chain constant region (as shown in figure 14), the constant region γ4 (as shown in SEQ ID NO. 14) and κ(as shown in SEQ ID NO. 17) of human (the vector atlas of pTSE as shown in figure 14, the preparation process as shown in the description page 3 section 0019 of CN103525868A). Transient transfected HEK293E cells with the cloned vector were used to express antibodies. The antibody proteins were purified with protein A affinity column by the AKTA.

### 2. The determination of the affinity of The monoclonal Antibody with BIAcore X100

The affinities of the antibodies were determined by ligand- capture method. Anti-human IgG was coupled to the surface of CM5 chip, and DFPD1-9, DFPD1-10, DFPD1-11, DFPD1-12 and DFPD1-13 were diluted respectively to ensure the capturing about 300 RU of the antibody by the anti-human IgG. A series of concentration gradient of the PD-1 (1000 nM, 500 nM, 250 nM, 125 nM, 62.5 nM, 31.25 nM, 15.625 nM, 7.8125 nM, 3.9063 nM, 1.9531 nM and 0.9766 nM) flowed through the surface of the stationary phase to determine the affinities of the antibodies. The results show the affinity of the antibodies has no obvious difference (as shown in table 2).

**TABLE 2**

| Determination of The Affinity Constants For The Anti-PD 1 Full-length Antibody | | | |
|---|---|---|---|
| Sample | ka(1/Ms) | kd(1/s) | KD |
| DFPD1-9 | 1.626E+4 | 1.045E-4 | 6.429E-9 |
| DFPD1-10 | 3.285E+4 | 1.300E-4 | 3.957E-9 |
| DFPD1-11 | 9.357E+3 | 1.015E-4 | 1.085E-8 |
| DFPD1-12 | 1.327E+4 | 2.975E-4 | 2.242E-8 |
| DFPD1-13 | 1.811 E+4 | 1.079E-4 | 9.504E-9 |

### 3. The Binding Assay of The anti-PD-1 Antibody

PD-1-His in pH9.6 carbonate buffer solution, 60 ng/well / 100 µl, was used for coating 96well plate at 4 °C overnight. After washing five times with 300µl /well PBST, the wells were blocked for two hours with 1% BSA-PBS at 37 °C. Different dilution of the antibodies DFPD1-9, DFPD1-10, DFPD1-11, DFPD1-12 and DFPD1-13 were added. The highest concentration of these five kinds of antibodies is 16 µg/mL, diluted for 4 times to 11 gradients, and the last well was used as the negative control which was added PBS diluent only. After incubating at 37 °C for one hour, the wells were washed five times with 300 µL /well of PBST, then adding the anti-human Fc-HRP secondary antibodies diluted at 1:40000 with 1 % BSA-PBS, incubating at 37 °C for one hour. After staining with TMB stain solution kit, 100 µL /well, for 8 minutes at room temperature, the reaction was stopped with 50µL of 2 mol/L H₂SO₄/well, and the optical density was determined at 450 nm and 630 nm wavelength. The result is shown in figure 15, all antibodies can bind with PD-1.

### 4. The Antibody Competitive Inhibition Test Of PD-L1 Binding With PD-1

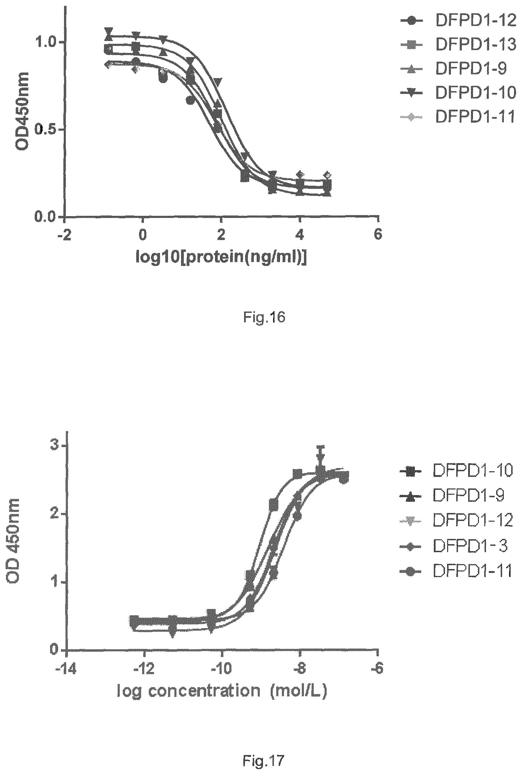

PDL1-Fc in pH9.6 carbonate buffer solution was used for coating plates at 4 °C overnight. After washing five times with PBST, the wells were blocked for two hours with 1 % BSA-PBS at 37 °C. The following five antibodies with 4 µg/ml PD1-His, DFPD1-9, DFPD1-10, DFPD1-11, DFPD1-12 and DFPD1-13 were diluted respectively, starting from the molar ratio 10:1 of the antibody and PD1-His, the gradient diluting for five times and 9 dilution gradient of each sample. After incubating at 37 °C for one hour, the wells were washed five times with PBST, and then the mouse anti-His antibody HRP labeled with 1% BSA-PBS diluted were added. After incubating at 37 °C for one hour, the wells were stained with TMB stain solution kit, 100 µl/well, for 8 minutes at room temperature. The reaction was stopped with 50µl of 10% H₂SO₄/well. The optical density was read at 450 nm and 630 nm wavelength. The result is shown in figure 16, DFPD1-9, DFPD1-10, DFPD1-11, DFPD1-12 and DFPD1-13 can inhibit the binding of PD-1 and PD-L1.

### EXAMPLE 6

### The Binding Assay of Anti-PD-1 Antibody and Cell-Surface PD-1

Firstly, the CHO cell line with stable PD-1 overexpression was made and named PD1-CHO. After coating 96-well plates with gelatin, PD1-CHO cells was digested by trypsin and then stopped. After centrifuging and suspending, the cells were diluted to 2×10⁵ cells/ml, 100 µL per well in 96-well plates, totally 12 well x 6 row, namely 2 x 10⁴ cells/well, and cultured at 5% CO₂, 37 °C overnight. The culture medium were discarded the next day, and the wells were washed one time with 350 µl precooling PBS. Freshly prepared 2% PFA was added to fix 5 minutes, and then PBS was used to wash two times.

The full-length anti-PD-1 antibody of double dilution was added into the cell plates, the diluent is PBS with 0.5% BSA. Sample concentration started from 100 µg/mL, diluting for 8 times, totally 12 gradients of dilution. After incubating for 30 minutes at room temperature, supernatant was discarded, and wells were washed wells three times with 350 µL pre-cooled PBS. The anti-human Fc-HRP Secondary antibodies diluted at 1:5000 were added, and incubated for 15 minutes at room temperature. After washing three times with 350 µL PBS, 100 µL TMB stain solution was added into each well, staining for 15 to 30 minutes at room temperature. After adding 50 µL 2mol/L H₂SO₄ per well to stop the staining, the optical density was read at 450 nm and 630nm wavelength with the microplate reader. The results were processed with Graph pad prism software, and the binding constant was calculated (as shown in figure 17).

The invention includes all combinations of the recited particular embodiments. Further embodiments and the full scope of applicability of the invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description. All publications, patents, and patent applications cited herein, including citations therein, are hereby incorporated by reference in their entirety for all purposes.

## Claims

1. An anti-PD-1 monoclonal antibody, **characterized in** comprising light chains and heavy chains, wherein the light chain contains three complementarity determining regions (CDRs) which are named as LCDR1, LCDR2 and LCDR3; the LCDR1 comprises one of peptides whose amino acid sequences are shown as of SEQ ID NO. 18, SEQ ID NO. 19, SEQ ID NO. 20, SEQ ID NO. 21, SEQ ID NO. 22, SEQ ID NO. 23 or SEQ ID NO. 24; the LCDR2 comprises one of the peptide whose amino acid sequences are shown as SEQ ID NO. 25, SEQ ID NO. 26, SEQ ID NO. 27, SEQ ID NO. 28, SEQ ID NO. 29 or SEQ ID NO. 30; the LCDR3 comprises one of the peptides whose amino acid sequences shown as SEQ ID NO. 31, SEQ ID NO. 32, SEQ ID NO. 33, SEQ ID NO. 34, SEQ ID NO. 35, SEQ ID NO. 36 or SEQ ID NO. 37.

2. The anti-PD-1 monoclonal antibody according to claim 1, wherein the light chain contains a light chain variable region that is selected from one of the peptides having amino acid sequences shown as SEQ ID NO.5, SEQ ID NO.6, SEQ ID NO.7, SEQ ID NO. 8, SEQ ID NO.9, SEQ ID NO.10 or SEQ ID NO.11.

3. An anti-PD-1 monoclonal antibody, **characterized in** comprising light chains and heavy chains, wherein the heavy chain contains three complementarity determining regions which are named as HCDR1, HCDR2 and HCDR3; the HCDR1 comprises one of peptides whose amino acid sequences are shown as SEQ ID NO. 38 or SEQ ID NO. 39; the HCDR2 comprises one of peptides whose amino acid sequences are shown as SEQ ID NO. 40, SEQ ID NO. 41 or SEQ ID NO. 42; the HCDR3 comprises one of peptides whose amino acid sequences are shown as SEQ ID NO. 43 or SEQ ID NO. 44.

4. The anti-PD-1 monoclonal antibody according to claim 3, wherein the heavy chain contains a heavy chain variable region that is selected from one of the peptides having amino acid sequences shown as SEQ ID NO.1, SEQ ID NO.2, SEQ ID NO.3 or SEQ ID NO. 4.

5. An antibody, a polypeptide or a protein, wherein the antibody, the polypeptide or the protein contains light chains or heavy chains according to any one of claims 1 to 4.

6. A polynucleotide or combination of polynucleotides, wherein the polynucleotide sequence or the combination encodes light chains or heavy chains according to any one of claims 1 to 4.

7. A recombinant DNA expression vector, wherein the recombinant DNA expression vector contains a polynucleotide sequence or combination according to claim 6.

8. A host cell, wherein the host cell is used when transfecting the recombinant DNA expression vector, and the host cell is selected from prokaryotic cells, yeasts, insects or mammalian cells.

9. The anti-PD-1 monoclonal antibody according to any one of claims 1 to 4, wherein the anti-PD-1 monoclonal antibody is a human antibody; the heavy chain having a constant region selected from IgG1, IgG2, IgG3 or IgG4; the light chain having a constant region that is Cκ or C_{λ}.

10. A whole antibody, a single-chain antibody, a single domain antibody, a bi-specific antibody, a drug-conjugated antibody or chimeric antigen receptor T-cell immunotherapy, containing light chains or heavy chains according to any one of claims 1 to 4 .

11. A monoclonal antibody, an artificial vector, a drug or a combination of drugs thereof, wherein the monoclonal antibody, the artificial vector, the drug or the combinations of drugs contains light chains or heavy chains according to any one of claims 1 to 4.

12. A detection reagent or a kit thereof, wherein the detection reagent or the kit contains light chains or heavy chains according to any one of claims 1 to 4.

## Amended claims

### Amended claims under Art. 19.1 PCT

**1.** An anti-PD-1 monoclonal antibody, **characterized in** comprising light chains and heavy chains, wherein the light chain contains three complementarity determining regions (CDRs) which are named as LCDR1, LCDR2 and LCDR3; the LCDR1 comprises one of peptides whose amino acid sequences are shown as of SEQ ID NO. 18, SEQ ID NO. 19, SEQ ID NO. 20, SEQ ID NO. 21, SEQ ID NO. 22, SEQ ID NO. 23 or SEQ ID NO. 24; the LCDR2 comprises one of the peptide whose amino acid sequences are shown as SEQ ID NO. 25, SEQ ID NO. 26, SEQ ID NO. 27, SEQ ID NO. 28, SEQ ID NO. 29 or SEQ ID NO. 30; the LCDR3 comprises one of the peptides whose amino acid sequences shown as SEQ ID NO. 31, SEQ ID NO. 32, SEQ ID NO. 33, SEQ ID NO. 34, SEQ ID NO. 35, SEQ ID NO. 36 or SEQ ID NO. 37.

**2.** The anti-PD-1 monoclonal antibody according to claim 1, wherein the light chain contains a light chain variable region that is selected from one of the peptides having amino acid sequences shown as SEQ ID NO.5, SEQ ID NO.6, SEQ ID NO.7, SEQ ID NO. 8, SEQ ID NO.9, SEQ ID NO.10 or SEQ ID NO.11.

**3.** An anti-PD-1 monoclonal antibody, **characterized in** comprising light chains and heavy chains, wherein the heavy chain contains three complementarity determining regions which are named as HCDR1, HCDR2 and HCDR3; the HCDR1 comprises one of peptides whose amino acid sequences are shown as SEQ ID NO. 38 or SEQ ID NO. 39; the HCDR2 comprises one of peptides whose amino acid sequences are shown as SEQ ID NO. 40, SEQ ID NO. 41 or SEQ ID NO. 42; the HCDR3 comprises one of peptides whose amino acid sequences are shown as SEQ ID NO. 43 or SEQ ID NO. 44.

**4.** The anti-PD-1 monoclonal antibody according to claim 3, wherein the heavy chain contains a heavy chain variable region that is selected from one of the peptides having amino acid sequences shown as SEQ ID NO.1, SEQ ID NO.2, SEQ ID NO.3 or SEQ ID NO. 4.

**5.** An antibody, a polypeptide or a protein, wherein the antibody, the polypeptide or the protein contains light chains or heavy chains according to any one of claims 1 to 4.

**6.** A polynucleotide or combination of polynucleotides, wherein the polynucleotide sequence or the combination encodes light chains or heavy chains according to any one of claims 1 to 4.

**7.** A recombinant DNA expression vector, wherein the recombinant DNA expression vector contains a polynucleotide sequence or combination according to claim 6.

**8.** A host cell transfecting the recombinant DNA expression vector of claim 7, wherein the host cell is selected from prokaryotic cells, yeasts, insects or mammalian cells.

**9.** The anti-PD-1 monoclonal antibody according to any one of claims 1 to 4, wherein the anti-PD-1 monoclonal antibody is a human antibody; the heavy chain having a constant region selected from IgG1, IgG2, IgG3 or IgG4; the light chain having a constant region that is Cκ or C_{λ}.

**10.** A whole antibody, a single-chain antibody, a single domain antibody, a bi-specific antibody, a drug-conjugated antibody, containing light chains or heavy chains according to any one of claims 1 to 4.

**11.** A monoclonal antibody, an artificial vector, a drug or a combination of drugs thereof, wherein the monoclonal antibody, the artificial vector, the drug or the combinations of drugs contains light chains or heavy chains according to any one of claims 1 to 4.

**12.** A detection reagent or a kit thereof, wherein the detection reagent or the kit contains light chains or heavy chains according to any one of claims 1 to 4.
